Europäisches Patentamt

European Patent Office

Office européen des brevets

(19)

(11) Publication number: **0 061 217**
**A2**

(12) **EUROPEAN PATENT APPLICATION**

(21) Application number: 82200266.3

(22) Date of filing: 03.03.82

(51) Int. Cl.³: **A 61 K 9/24**
**A 61 K 9/52, A 61 K 9/54**
**A 61 K 31/19**

(30) Priority: 19.03.81 IT 2057881

(43) Date of publication of application:
29.09.82 Bulletin 82/39

(84) Designated Contracting States:
AT BE CH DE FR GB IT LI LU NL SE

(71) Applicant: PHARMATEC S.p.A.
Via Carducci, 35
I-20090 Trezzano Sul Naviglio Milano(IT)

(72) Inventor: Giudice, Vittorio, Dr.
Via Oldrado da Tresseno 2
I-20100 Milano(IT)

(72) Inventor: Ravelli, Vittorino, Dr.
Via Palmanova 189
I-20100 Milano(IT)

(74) Representative: de Pasquale, Carlo
Via Carlo Ravizza 53
I-20149 Milano(IT)

(54) Ibuprofen-containing sustained release pharmaceutical composition.

(57) The invention relates to an ibuprofen-containing sustained release pharmaceutical composition, comprising spheroids consisting of three components, namely a central core or microgranule of inert excipient, a layer of active principle applied on said central core, and an outer coating of active excipient forming the sustained release membrane. With this composition a remarkable reduction of the release rate is obtained in comparison with the known administration forms, reproducible results are obtained as release patterns of the active principle versus time and possible side effects are avoided.

EP 0 061 217 A2

- 1 -

# IBUPROFEN-CONTAINING SUSTAINED RELEASE PHARMACEUTICAL COMPOSITION

The present invention relates to a sustained release pharmaceutical composition containing ibuprofen.

Ibuprofen is a drug belonging to the non steroid antiinflammatory category, having also a good analgesic activity.

Its chemical name according to the IUPAC nomenclature is $\alpha$-methyl-4-(2-methyl-propyl) benzene acetic acid; another equivalent chemical name is 2-(4-isobutylphenyl) propionic acid.

Administration of this drug through the oral route, in the conventional form such as sugar coated tablets, rapidly causes high blood levels to which the therapeutic effects are bound, but in view of the very short half life of this drug, also a very rapid fall of the blood level and consequently of the therapeutic efficiency.

For this reason, with the conventional administration forms for oral use, the therapeutic coverage arising from a drug blood level steadily above the minimum effective level, may be obtained only with frequent administrations combining to the high risk that the posology is not observed by the patient, the drawback of producing several times during the day peak blood level with possible side effects.

The production of tablets or pills with controlled sustained release, using the conventional techniques well known in the pharmaceutical field, does not allow to obtain reproducible results as to patterns of active principle release versus time.

This is mainly due to the low melting point of ibuprofen, further decreasing with formation of eutectics when it is blended with the usual excipients, causing a partial melting of ibuprofen in the tabletting stage, and so the release rate of the drug from the pharmaceutical form is scarcely reproducible.

The object of the present invention is a sustained release pharmaceutical composition consisting of a hard gelatine capsule containing spheroids having ibuprofen contents of 75-85% obtained by adding powdery ibuprofen on sugar-starch cores by means of suitable binders such as low viscosity polyvinylpyrrolidone and subsequently coated with high viscosity polyvinylpyrrolidone.

The term "spheroids" normally means, as used herein, spherical granules or pellets with a diameter of about 0,5-2 mm.

A type of low viscosity polyvinylpyrrolidone is for instance that available on the market under the trademark KOLLIDON 25$^{(R)}$ of the company BASF AKTIENGESELL-SCHAFT of Ludwigshafen, Federal Republic of Germany.

A type of high viscosity polyvinylpyrrolidone is for instance that available on the market under the trademark KOLLIDON 90$^{(R)}$ of the above quoted company BASF.

The reduction in the release rate is sharp, as compared with the tablet form. When using the method described in USP XX, page 959 for the dissolution rate of oral use forms, conveniently suited to the case, e decrease of about 50% in the release rate after 30 minuted is observed.

In comparison with the conventional administration form, releasing the entire active principle in one hour, the form of the present invention releases the active principle in four hours.

The sustained release composition of the present invention may be made by using well known techniques of pharmaceutical preparation.

For instance the spheroids may be made by using a classic confection pan in which the ibuprofen active principle is applied by rotation to the sugar-starch granules with the aid of the binder, and then treated again in a confection pan with the coating solution.

The so obtained spheroid are then dosed in hard gelatine capsules so as to obtain a dosage of 300 mg ibuprofen in sustained release capsular form by a conventional spheroid encapsulating apparatus.

The following is a non limiting example of preparation of the ibuprofen-containing sustained release pharmaceutical composition of the invention.

## EXAMPLE

21.0 kg of inert microgranules (having a composition of 80-90% sucrose and 10-20% corn starch) having a diameter less than 646 $\mu$ are placed in a confection pan.

The pan is being rotated at 20 rpm and the mass of inert microgranules is wetted with a 15% solution of KOLLIDON 25 in 95% ethyl alcohol and the powdery ibuprofen is sprinkled on the rotating wet mass.

These steps are repeated until 115.5 kg of powdery ibuprofen are applied, using a total amount of 10 kg of solution of KOLLIDON 25. The so obtained spheroids are dried in an air circulation oven at a temperature of 35ºC.

The so obtained spheroids are again placed in a confection pan which is being rotated at 10 rpm and 3 kg of a 20% solution of KOLLIDON 90 in 95% ethyl alcohol are added. The evaporation of solvent is first promoted with a hot air flow and thereafter the product is placed in an air circulation oven where it is completely dried at a temperature of 35°C.

The product obtained is screened through a wire with 1.7 mm mesh to remove possible aggregations. The final spheroids obtained are then dosed in hard gelatine capsules by using a conventional chronoid encapsulation apparatus, so as to obtain a dosage of 300 mg ibuprofen per capsule. In this way an ibuprofen-containing sustained release pharmaceutical composition of the invention is obtained, having the above mentioned release features.

A number of pharmaceutical and clinical studies were carried out to check the results obtained with the sustained release composition of the invention, and said results are briefly discussed hereinafter with reference to the accompanying drawings, in which:

Figs. 1 and 2 are diagrams of serum ibuprofen levels following single dose of 1200 mg standard or slow-release ibuprofen;

Fig. 3 is a diagram of serum ibuprofen levels during 60 hours continuous treatment with standard ibuprofen 400 mg t.d.s. (doses spaced 6 hourly) and slow-release ibuprofen 600 mg 12 hourly;

Figs. 4 and 5 are diagrams of serum ibuprofen levels following final dose of treatment with standard ibuprofen 400 mg t.d.s. or slow-release ibuprofen 600 mg b.d.;

Fig. 6 is a diagram of mean serum ibuprofen levels during study; and

Fig. 7 is a diagram of visual analogue pain scores.

A crossover study has been conducted in six healthy volunteers to compare the bioavailability, pharmacokinetics and tolerance of two oral formulations of ibuprofen, a slow-release formulation of the invention and the standard preparation.

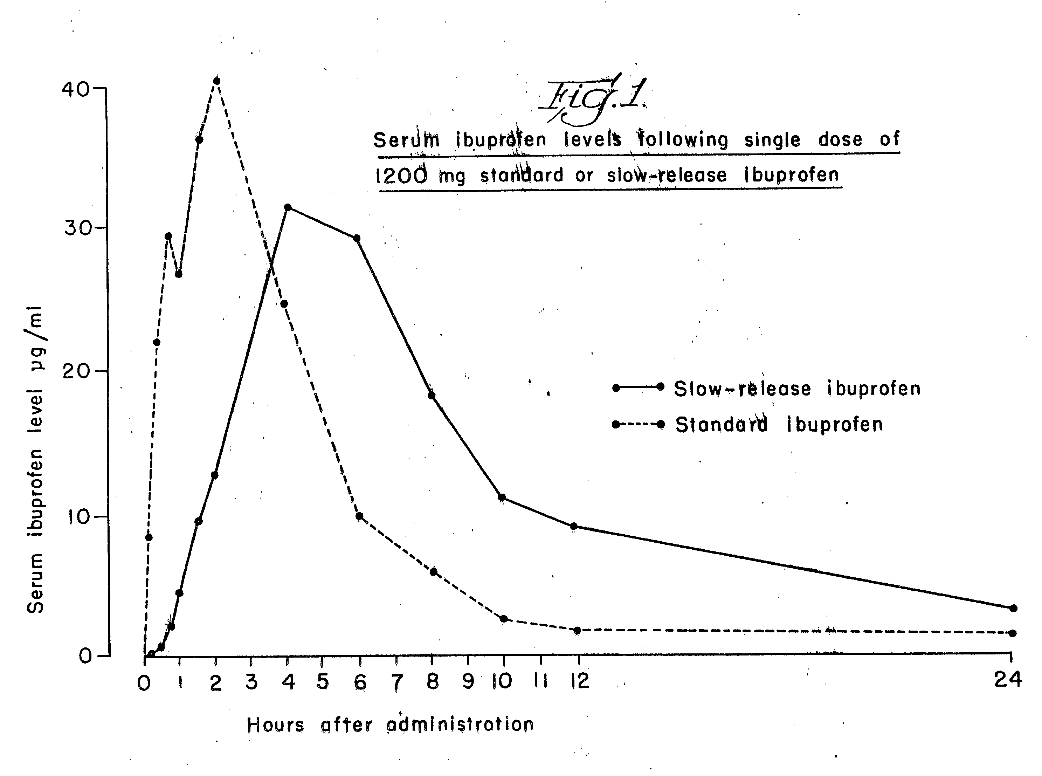

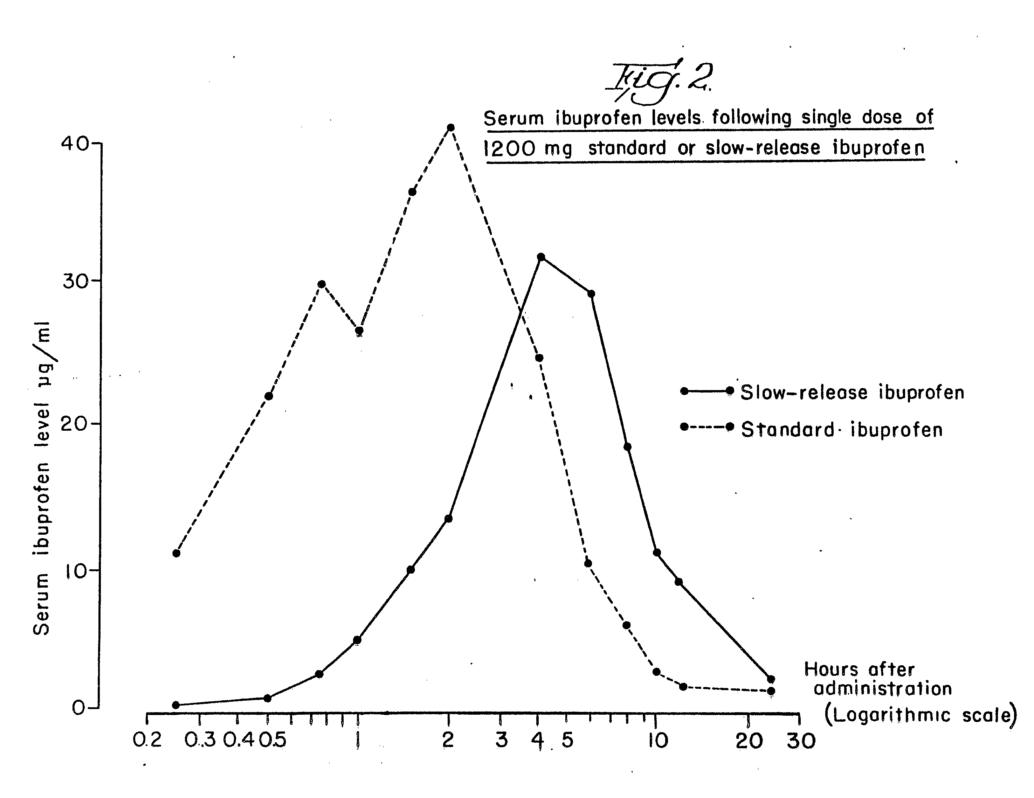

Following administration of a single dose of the slow-release formulation of ibuprofen, peak serum ibuprofen levels were achieved later and sustained for longer than with the same dose (1200 mg) of the standard preparation. Although the peak level achieved was greater with the standard preparation, the difference was relatively small and not statistically significant. The pattern of serum ibuprofen levels was in neither case appreciably different after three days' continuous treatment (see Figs. 1 and 2).

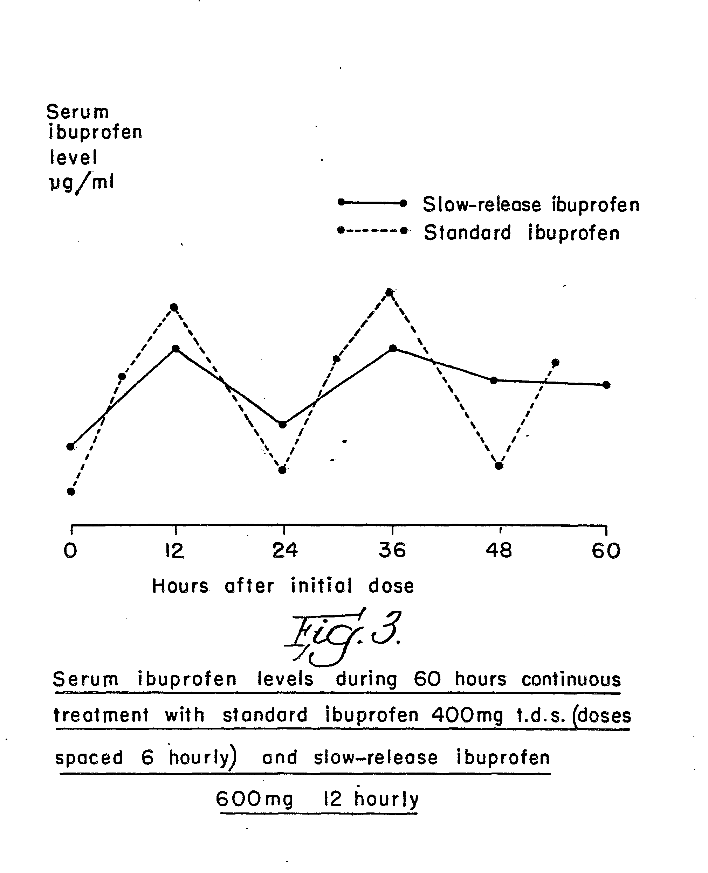

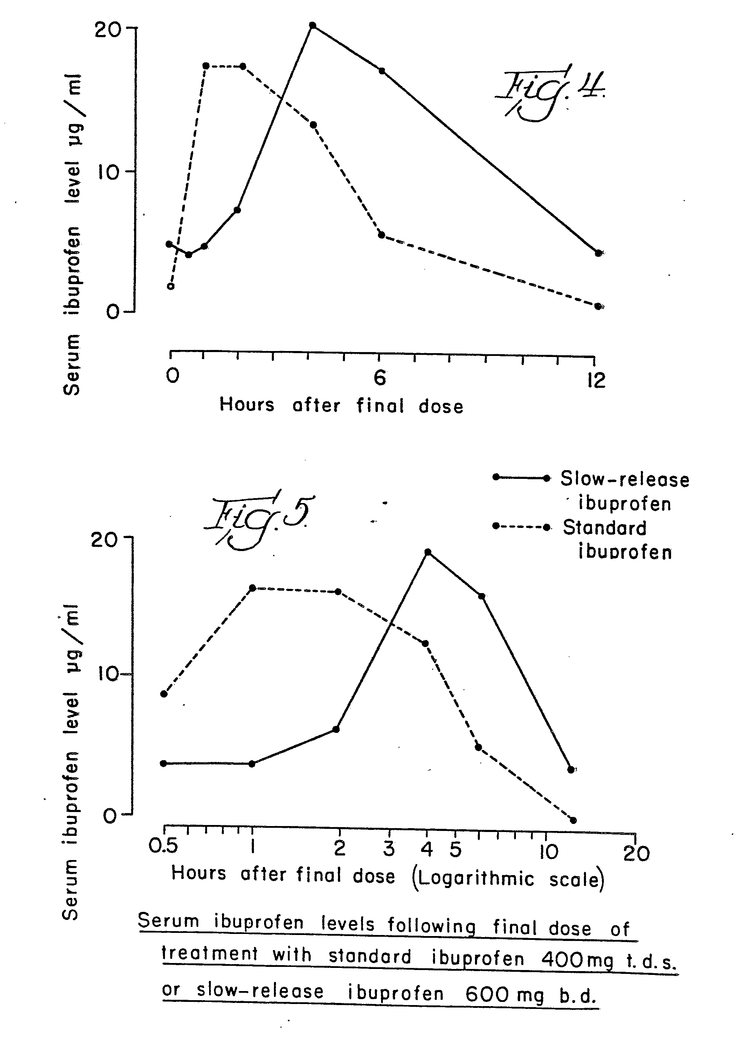

Treatment was continued for $2\frac{1}{2}$ days with standard ibuprofen 400 mg t.d.s.(6 hourly intervals during day) or slow-release ibuprofen 600 mg every twelve hours. The standard formulation resulted in large diurnal fluctuations, with serum levels falling to almost undetectable levels in the morning. With the slow-release formulation, fluctuations (about the same "average" serum level) were less and diminished progressively as treatment continued. By the third day it appeared that the difference between morning and evening levels has been eliminated, the minimum serum level being approximately equal to the average level achieved during the day with standard ibuprofen (see Figs. 3, 4 and 5).

No symptomatic adverse reaction were reported and an extensive series of laboratory screening investigations failed to show any toxic effects.

Although in this study continuous treatment only extended over a three day period, there is very indication that the slow-release formulation of ibuprofen offers definite advantages over the standard formulation, combining a reduced number of daily doses with better sustained blood levels of the drug. To have a confirmation of this assumption, it was considered desirable to conduct an extended study in order to confirm that no accumulation of the drug was occurring during long term treatment.

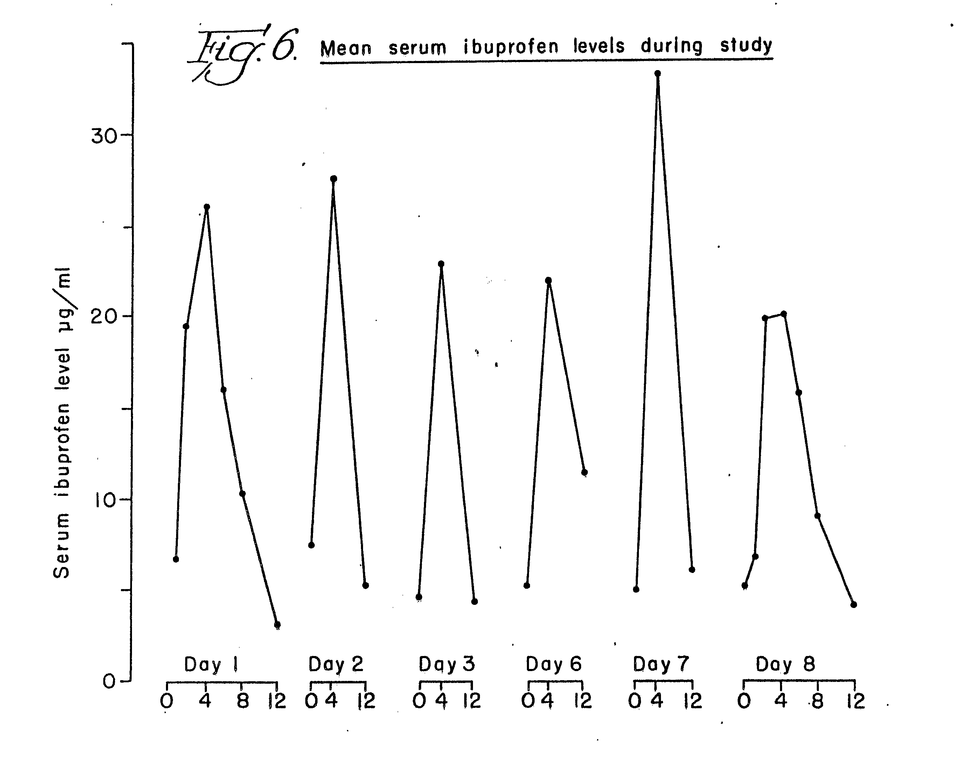

The results obtained on a certain number of subjects show similar pharmacokinetic profile to those obtained in the earlier study but additionally serve to confirm that accumulation of the drug does not occur during continued administration. In particular, it should be noted that serum ipubrofen levels fell to very similar figures prior to the morning dose of the drug throughout the eight days of the study (Fig. 6).

This study demonstrates that the pharmacokinetic profile of slow-release ipubrofen is not appreciably altered after 8 days' continuous treatment and that there is no tendency for the drug to accumulate during this period.

A clinical trial has then been conducted in 54 patients with osteoarthritis to compare the efficacy and tolerance of three weeks treatment with either the standard formulation of ibuprofen 400 mg t.d.s. (25 patients) or the sustained-release (SR) formulation of ibuprofen of the invention in a dosage of 600 mg b.d. (29 patients).

Weekly assessments by the investigator and daily self-assessment by patients was employed, embracing several parameters of efficacy and tollerance.

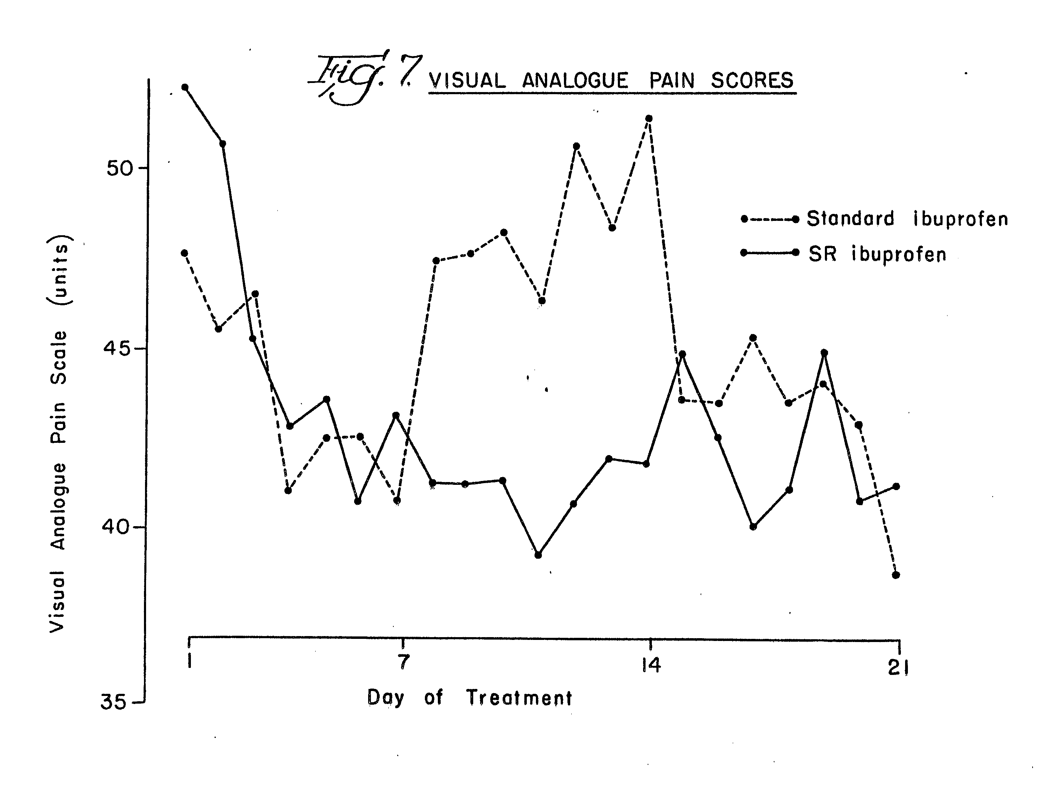

Most of the parameters of efficacy (relating to pain, joint stiffness, joint movement, etc.) considered showed small progressive improvements with standard ibuprofen and somewhat greater improvements with the SR formulation. A number of the changes during treatment were statistically significant with SR ibuprofen treatment but not with standard ibuprofen (see Fig. 7).

For none of the parameters was there a significant difference between the changes during treatment seen in the two treatment groups. It must be realised, however, that the magnitude of change seen, the scatter of these changes and the number of patients showing changes is such that firm conclusion cannot be drawn regarding statistical equivalence of the two formulations.

There was little apparent difference in tolerance to the two treatment groups. Gastrointestinal side effects were seen in five patients receiving standard ibuprofen (20%) and four (14%) receiving SR ibuprofen, necessitating the withdrawal of three patients from the study (two standard, one SR). One further patient receiving standard

0061217

ibuprofen was withdrawn because of the development of a rash. No other major side effects were seen with either formulation.

In conclusion, the results indicate that with SR ibuprofen treatment, there were statistically significant improvements in a number of indices of efficacy in patients with osteoarthritis and suggest that it is at least as efficacious and well tolerated as the standard formulation of the drug. Under these circumstances, the simplified treatment regimes possible with the SR preparation can be considered an advantage in clinical practice.

CLAIMS

1) Ibuprofen-containing sustained release pharmaceutical composition characterized by the fact of comprising spheroids consisting of three components; namely a central core or microgranule of inert excipient, a layer of active principle applied on said central core, and an outer coating of active excipient forming the sustained release membrane.

2) Ibuprofen-containing sustained release pharmaceutical composition according to Claim 1, characterized by the fact that the layer of active principle is applied on the inert microgranules by adding it as a powder with the aid of a solution of a suitable binder.

3) Ibuprofen-containing sustained release pharmaceutical composition according to Claim 1, characterized by the fact that the spheroids are spherical granules with a diameter of about 0.5 - 2 mm.

4) Ibuprofen-containing sustained release pharmaceutical composition according to Claim 1, characterized by the fact that the inert microgranules are sugar and starch cores.

5) Ibuprofen-containing sustained release pharmaceutical composition according to Claim 1, characterized by the fact that the composition of the inert microgranules is 80-90% sucrose and 10-20% corn starch.

6) Ibuprofen-containing sustained release pharmaceutical composition according to Claim 1, characterized by the fact of having an ibuprofen contents of 75-85%.

7) Ibuprofen-containing sustained release pharmaceutical composition according to Claim 2, characterized by the fact that the binder is a 15% solution of low viscosity polyvinylpyrrolidone in 95% ethyl alcohol, and the solvent is evaporated after application of the powdery active principle.

8) Ibuprofen-containing sustained release pharmaceutical composition according to Claim 1, characterized by the fact that the outer coating active excipient consists of high viscosity polyvinylpyrrolidone, which is applied as a 20% solution in 95% ethyl alcohol, the solvent being then evaporated.

9) Ibuprofen-containing sustained release pharmaceutical composition according to Claim 1, characterized by the fact that the spheroids are dosed in hard gelatine capsules as an oral administration form.

10) Ibuprofen-containing sustained release pharmaceutical composition according to Claim 9, characterized by the fact that each hard gelatine capsule contains a dosage of 300 mg of active principle.

FIG.1.

Serum ibuprofen levels following single dose of 1200 mg standard or slow-release ibuprofen

Slow-release ibuprofen
Standard ibuprofen

Serum ibuprofen level µg/ml

Hours after administration

Fig. 2

Serum ibuprofen levels following single dose of
1200 mg standard or slow-release ibuprofen

Slow-release ibuprofen
Standard ibuprofen

Serum ibuprofen level µg/ml

Hours after
administration
(Logarithmic scale)

0061217

Serum
ibuprofen
level
µg/ml

●————● Slow-release ibuprofen
●------● Standard ibuprofen

0   12   24   36   48   60

Hours after initial dose

*Fig. 3.*

Serum ibuprofen levels during 60 hours continuous
treatment with standard ibuprofen 400mg t.d.s. (doses
spaced 6 hourly) and slow-release ibuprofen
600mg 12 hourly

Fig. 4

Fig. 5

Serum ibuprofen levels following final dose of treatment with standard ibuprofen 400 mg t.d.s. or slow-release ibuprofen 600 mg b.d.

Fig. 6. Mean serum ibuprofen levels during study

Fig. 7 VISUAL ANALOGUE PAIN SCORES

Standard ibuprofen
SR ibuprofen

Visual Analogue Pain Scale (units)

Day of Treatment